(19) Europäisches Patentamt
European Patent Office
Office européen des brevets

(11) Veröffentlichungsnummer: **0 477 552 B1**

# (12) EUROPÄISCHE PATENTSCHRIFT

(45) Veröffentlichungstag der Patentschrift: **16.08.95**

(51) Int. Cl.[6]: **A61K 31/365**

(21) Anmeldenummer: **91114331.1**

(22) Anmeldetag: **27.08.91**

## (54) Verwendung von 10-Ring-Lactonen als Lipidregulatoren.

(30) Priorität: **28.08.90 DE 4027100**

(43) Veröffentlichungstag der Anmeldung:
**01.04.92 Patentblatt 92/14**

(45) Bekanntmachung des Hinweises auf die Patenterteilung:
**16.08.95 Patentblatt 95/33**

(84) Benannte Vertragsstaaten:
**BE DE DK ES FR GB GR IT LU NL**

(56) Entgegenhaltungen:
**EP-A- 0 333 024**

**J. ORG., CHEM. Band 44, Nr. 12, 1979, Seiten 2008-2012; T. WAKAMATSU et al.: "Naturally occurring ten-membered lactones: Total synthesis of (+)-diplodialide A, (+)-diplodialide C, and (+)-decan-9-olide"**

(73) Patentinhaber: **HOECHST AKTIENGESELLSCHAFT**

**D-65926 Frankfurt am Main (DE)**

(72) Erfinder: **Granzer, Ernold, Dr. Dr.**
**Falkensteiner Strasse 24**
**W-6233 Kelkheim/Ts. (DE)**
Erfinder: **Hammann, Peter, Dr.**
**Bürgermeister-Rühl-Strasse 20**
**W-6113 Babenhausen (DE)**
Erfinder: **Wink, Joachim, Dr.**
**Magdeburger Strasse 14**
**W-6074 Rödermark (DE)**
Erfinder: **Grabley, Susanne, Dr.**
**Hölderlinstrasse 7**
**W-6240 Königstein/Ts. (DE)**

**EP 0 477 552 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Erhöhte Serum-Cholesterin-Konzentrationen allein, oder mit gleichzeitig erhöhten Serum-Triglyceriden stellen einen Risikofaktor für die Arteriosklerose dar, die sich vor allem in Form der Koronarsklerose, des Herzinfarktes, peripherer Gefäß-Verschlüsse, aber auch in Form von Apoplexie im Bereich des Gehirns manifestieren kann. Andererseits ist aus zahlreichen Studien bekannt, daß eine Normalisierung bzw. Senkung erhöhter Serum-Cholesterin-Spiegel dieses Risiko entscheidend mindern kann. Dies kann in beschränktem Maße durch diätetische Maßnahmen und in stärkerem Ausmaße durch eine medikamentöse Therapie erfolgen. Ebenfalls ist es möglich, bereits vorhandene arteriosklerotische Gefäßveränderungen, sofern sie nicht bereits in ein irreparables Stadium (direkte Verkalkung) übergegangen sind, durch massive Verminderung eines sehr stark erhöhten Serum-Totalcholesterins zur Rückbildung zu bringen (Regression).

In der westlichen Welt, mit ihrem über Jahrzehnte andauernden diätetischen Fehlverhalten, sind Todesfälle in der Bevölkerung mittelbar oder unmittelbar auf die Arteriosklerose in nahezu 50 % der Fälle oder mehr zurückzuführen. Es ist daher äußerst erwünscht, hochwirksame Medikamente zur Verminderung von Serum-Cholesterin zu entwickeln, die gleichzeitig gut verträglich sind. In der Vergangenheit standen hierfür Medikamente vom Typ der Fibrate, der Nikotinsäure, der nicht resorbierbaren GallensäureSequestrantien, Probucol und, vor allem in den letzten Jahren, der kompetitiven HMG-CoA-Reduktase-Inhibitoren z.B. vom Typ des Lovastatin zur Verfügung (Endo, A.J., J. Med. Chem. 28, 401 (1985)).

Es wurde nun überraschend gefunden, daß 10-Ring-Lactone den Serum-Cholesterin Spiegel herabsetzen können.

Die Erfindung betrifft somit die Verwendung der Verbindung der allgemeinen Formel I,

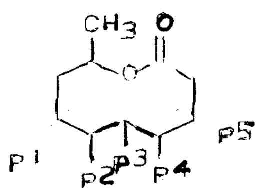

I

in der unabhängig voneinander
$R^1$ und $R^4$ Wasserstoff und Hydroxyl
$R^2$ und $R^3$ Hydroxyl,
$R^5$ Wasserstoff, Hydroxyl und eine Oxogruppe sind und
$R^2$ und $R^3$ sowie $R^3$ und $R^4$ jeweils gemeinsam eine Doppelbindung bilden können,
zur Herstellung eines Arzneimittels zur Senkung des Serum-Cholesterins.

Im folgenden wird die Erfindung detailliert, insbesondere in ihren bevorzugten Ausführungsformen, beschrieben. Ferner wird die Erfindung durch den Inhalt der Ansprüche bestimmt.

Die Gewinnung der Verbindung der allgemeinen Formel I ist in EP 333 024 beschrieben. Penicillium-Stämme, bevorzugt DSM 4209 und 4210, synthetisieren diese Verbindung in herkömmlichen Fermentationslösungen. Durch Extraktion von Mycel und Kulturbrühe mit organischen Lösungsmitteln und anschließender chromatographischer Reinigung können sie isoliert werden.

Die Verbindung der allgemeinen Formel I besitzt hypolipämische Eigenschaften. Die atherogenen Lipoproteine VLDL und LDL werden durch die Verbindung im Serum stark herabgesetzt, während die Wirkung auf das vasoprotektive HDL nur schwach ist. Auf diese Weise wird der vasoprotektive Index HDL-Cholesterin : LDL-Cholesterin stark erhöht.

Die hypolipämische Wirkung der Verbindung der allgemeinen Formel I konnte in folgender Untersuchung festgestellt werden:
Männliche Ratten mit einem Ausgangsgewicht von über 180 g erhielten täglich einmal (morgens) das Prüfpräparat der allgemeinen Formel I in Polyäthylenglykol 400 per Schlundsonde (0,5 ml/100 g Körpergewicht); die jeweilige Kontrollgruppe erhielt nur die gleiche Menge Lösungsmittel. Die letzte (7.) Applikation erfolgte 24 Stunden vor Blutentnahme und Tötung. Zu Futter und Wasser bestand freier Zugang während des Versuches. 24 Stunden vor der Blutentnahme, die retroorbital unter leichter Äthernarkose vor und nach der Behandlungsperiode (also am 1. und 8. Tag) erfolgte, wurde das Futter entzogen. Im Serum jedes einzelnen Tieres wurde Serum-Glutamat-Oxalat-Transaminase (SGOT), Serum-Glutamat-Pyruvat-Transaminase (SGPT), alkalische Phosphatase (aP), Bilirubin und Kreatinin bestimmt. Aus dem Serumpool aller Tiere einer Gruppe wurde als Maß für die Triglyceride, das Gesamtglycerin gemessen (Eggstein, M. Kreutz, F.H.: Klin. Wschr. 44, 262 and 267 (1966); Wahlefeld, A.W., In: H.O. Bergmeier: Methoden der enzymatischen

Analyse 2. Auflage, Band II, Verlag Chemie 1974, S. 1878). Unmittelbar nach der Blutentnahme wurden die Tiere durch Distorsion der Wirbelsäule getötet. Es wurden die Lebern entnommen und das relative Lebergewicht bestimmt. Außerdem wurde die Körpergewichtsentwicklung und der Futterverbrauch untersucht. Die statistische Auswertung erfolgte im t-Test nach Student.

Zur Analyse der Serum-Lipoproteine wurde nach der Behandlungsperiode das Serum aller Ratten einer Gruppe gepoolt. Die Serum-Lipoproteine wurden mit der präparativen Ultrazentrifuge (Kontron TGA, Rotor Beckmann 50.4 Ti) getrennt.

Die Flotation der Serum-Lipoproteine (Koga, S., Horwith, D.L., and Scanu, A.M.: Journal of Lipid Research 10, 577 (1969), Havel, R.J., Eder, H.A., and Bragdon, H.H: J. Clin. Invest. 34, 1345 (1955)) erfolgte bei folgenden Dichten:

| | |
|---|---|
| 1. VLDL | Dichte $<1{,}006$ |
| 2. LDL | Dichte 1,006 bis 1,04 |
| 3. HDL | Dichte 1,04 bis 1,21 |

Zur enzymatischen Bestimmung des Cholesterins nach der CHODPAP high performance-Methode (Siedel, J. Schlumberger, H., Klose, S., Ziegenhorn, J., and Wahlefeld, A.W.: J. Clin. Chem. Clin. Biochem. 19, 838 (1981)) sowie der Triglyceride nach der vollenzymatischen Bestimmung (Eggstein, M. Kreutz, F.H.: Klin. Wschr. 44, 262 and 267 (1966); Wahlefeld, A.W., In: H.O. Bergmeier: Methoden der enzymatischen Analyse 2. Auflage, Band II, Verlag Chemie 1974, S. 1878) in den getrennten Lipoproteinfraktionen wurden Testkombinationen von Boehringer/Mannheim verwendet; die Bestimmung des Proteins erfolgte nach der Methode von Lowry et al. (Lowry, O.H., Roseborough, N.J., Farr, A.L., J. Biol. Chem. 193, 265 (1951)).

Die Ergebnisse für die bevorzugte Verbindung der Formel I, in der $R^1$, $R^4$ und $R^5$ Hydroxyl bedeuten sowie $R^2$ und $R^3$ gemeinsam eine Doppelbindung bilden, sind in den Tabellen 1 bis 3 in den Beispielen aufgeführt.

Die Verbindung der allgemeinen Formel I kann zur Prophylaxe und Behandlung von Krankheiten verwendet werden, die auf einem erhöhten Cholesterinspiegel beruhen, insbesondere koronare Herzkrankheiten, Arteriosklerose und ähnlicher Krankheiten. Die Erfindung betrifft auch pharmazeutische Zubereitungen der Verbindung der Formel I.

Bei der Herstellung von Arzneimitteln können neben dem Wirkstoff auch pharmazeutisch unbedenkliche Zusatzstoffe, wie Verdünnungsmittel und/oder Trägermaterialien, verwendet werden. Hierunter sind physiologisch unbedenkliche Substanzen zu verstehen, die nach Vermischen mit dem Wirkstoff diesen in eine für die Verabreichung geeignete Form bringen.

Geeignete feste oder flüssige galenische Zubereitungsformen sind beispielsweise Tabletten, Dragees, Pulver, Kapseln Suppositorien, Sirupe, Emulsionen, Suspensionen, Tropfen oder injizierbare Lösungen sowie Präparate mit protrahierter Wirkstoff-Freigabe. Als häufig verwendete Trägerstoffe bzw. Verdünnungsmittel seien z.B. verschiedene Zucker oder Stärkearten, Cellulosederivate, Magnesiumcarbonat, Gelatine, tierische und pflanzliche Öle, Polyethylenglykole, Wasser oder andere geeignete Lösungsmittel sowie wasserhaltige Puffermittel, die durch Zusatz von Glucose oder Salzen isotonisch gemacht werden können, genannt.

Außerdem können gegebenenfalls oberflächenaktive Mittel, Farb- und Geschmacksstoffe, Stabilisatoren, sowie Konservierungsmittel als weitere Zusatzstoffe in den erfindungsgemäßen Arzneimittelzubereitungen Verwendung finden. Es können auch pharmakologisch akzeptable polymere Träger, wie z.B. Polyphenylpyrolidone, oder andere pharmazeutisch akzeptable Additive, wie z.B. Cyclodextrin oder Polysaccharide, verwendet werden. Die Verbindungen können insbesondere auch mit Additiven, die Gallensäure binden,im Besonderen nicht toxischen, im Gastrointestinal-Trakt nicht resorbierbaren, basischen Anionen-Austauschern, kombiniert werden.

Die Präparate können oral, rektal oder parenteral verabreicht werden. Vorzugsweise können die Präparate in Dosierungseinheiten hergestellt werden; insbesondere Tabletten, Kapseln, Suppositorien, stellen Beispiele für geeignete Dosierungseinheiten dar. Jede Dosierungseinheit, insbesondere für die orale Applikation, kann bis zu 1000 mg, bevorzugt jedoch 10 bis 100 mg, des aktiven Bestandteiles enthalten. Jedoch können auch darüber oder darunterliegende Dosierungseinheiten verwendet werden, die gegebenenfalls vor Verabreichung zu teilen bzw. zu vervielfachen sind.

Gegebenenfalls können die Dosierungseinheiten für die orale Verabreichung mikroverkapselt werden, um die Abgabe zu verzögern, oder über einen längeren Zeitraum auszudehnen, wie beispielsweise durch Überziehen oder Einbetten des Wirkstoffs in Teilchenform in geeignete Polymere, Wachse oder dergleichen.

Die parenterale Verabreichung kann unter Verwendung flüssiger Dosierungsformen, wie steriler Lösungen und Suspensionen erfolgen, die für die intramuskuläre oder subcutane Injektion bestimmt sind. Derartige Dosierungsformen werden durch Lösen bzw. Suspendieren einer abgemessenen Wirkstoffmenge in einem geeigneten physiologisch unbedenklichen Verdünnungsmittel wie beispielsweise einem wäßrigen oder öligen Medium und Sterilisierung der Lösung bzw. der Suspension gegebenenfalls unter Mitverwendung von geeigneten Stabilisatoren, Emulgatoren und/oder Konservierungsmitteln und/oder Antioxidantien hergestellt.

Die orale Applikationsform ist, insbesondere unter dem Gesichtspunkt einer langen Therapiedauer, bevorzugt und stellt eine wesentliche Erleichterung in der Prävention und Therapie oben angeführter Krankheiten dar.

Die pharmazeutischen Präparate werden nach allgemein üblichen Verfahren hergestellt. Das Dosierungsschema kann vom Typ, Alter, Gewicht, Geschlecht und medizinischen Zustand des Patienten oder Risikogefährdeten abhängen.

Im folgenden wir die Erfindung anhand von Beispielen weitergehend erläutert.

Beispiele

Tabelle 1:

Ratte, männlich, Stamm: HOE WISKf (SPF 71),
Auftrennung der Lipoproteine durch Ultrazentrifugation,

| Verbindung | Dosis mg/kg /Tag | % Veränderung im Vergleich zur Kontrolle: Totalcholesterin VLDL | Totalcholesterin LDL | Totalcholesterin HDL | Protein VLDL | Protein LDL | Triglyceride VLDL | Cholesterin HDL/LDL | Cholesterin bezogen auf Kontrolle |
|---|---|---|---|---|---|---|---|---|---|
| Formel I: $R^1$, $R^4$, $R^5$ Hydroxyl, $R^2$ und $R^3$ Doppelbindung | 15 | -40 | -31 | -13 | -12 | -20 | -22 | 4,74 | 1,26 |
| Lovastatin | 10 | -32 | - 2 | -14 | -22 | -16 | -15 | 3,32 | 0,89 |
| Lovastatin | 50 | -52 | -26 | -15 | -40 | -19 | -38 | 4,29 | 1,14 |
| Lovastatin | 300 | -49 | + 3 | - 9 | -37 | + 5 | -34 | 3,33 | 0,89 |
| Clofibrat | 100 | -46 | -31 | -26 | + 5 | + 2 | + 2 | 4,03 | 1,08 |
| Kontrolle | | | | | | | | 3,75 | 1,00 |

Tabelle 2:

Ratte, männlich, Stamm: HOE WISKf (SPF 71),

| Zahl der Tiere pre/post | Verbindung | Dosis in mg/kg /Tag | Serumenzyme SGOT (U/L) Anfangswert $\bar{x}$ | s | SGOT (U/L) Endwert $\bar{x}$ | s | SGPT (U/L) Anfangswert $\bar{x}$ | s | SGPT (U/L) Endwert $\bar{x}$ | s | aP (U/L) Anfangswert $\bar{x}$ | s | aP (U/L) Endwert $\bar{x}$ | s | Bilirubin (µMol/L) Anfangswert $\bar{x}$ | s | Bilirubin (µMol/L) Endwert $\bar{x}$ | s | Kreatinin (µMol/L) Anfangswert $\bar{x}$ | s | Kreatinin (µMol/L) Endwert $\bar{x}$ | s |
|---|---|---|---|---|---|---|---|---|---|---|---|---|---|---|---|---|---|---|---|---|---|---|
| 5/ 5 | Formel I; $R^1$, $R^4$, $R^5$ Hydroxyl, $R^2$ und $R^3$ Doppelbindung | 15 | 41 | 2 | 56 | 12 | 29 | 3 | 25 | 5 | 359 | 21 | 247 | 43 | 2,8 | 0,4 | 2,0 | 0,4 | 67 | 5 | 64 | 7 |
| 5/ 5 | Lovastatin | 10 | 42 | 6 | 59 | 9 | 31 | 4 | 25 | 9 | 409 | 57 | 302 | 17 | 3,8 | 0,4 | 2,7 | 0,4 | 60 | 7 | 65 | 4 |
| 5/ 5 | Lovastatin | 50 | 38 | 3 | 89 | 57 | 31 | 7 | 46 | 16 | 355 | 23 | 324 | 148 | 2,9 | 0,2 | 2,5 | 0,2 | 60 | 6 | 62 | 7 |
| 5/ 5 | Lovastatin | 300 | 59 | 11 | 107 | 52 | 32 | 5 | 44 | 18 | 386 | 75 | 369 | 74 | 2,7 | 0,6 | 2,9 | 0,6 | 64 | 2 | 61 | 5 |
| 20/19 | Kontrolle | | 55 | 14 | 69 | 10 | 31 | 5 | 29 | 6 | 384 | 61 | 296 | 52 | 2,9 | 0,6 | 3,2 | 0,6 | 65 | 4 | 71 | 6 |

$\bar{x}$ = Mittelwert

s = Standardabweichung

Tabelle 3:

Ratte, männlich, Stamm: HOE WISKf (SPF 71)

| Zahl der Tiere pre/ post | Verbindung | Dosis in mg/kg /Tag | % Veränderung versus Kontrolle: Körpergewicht post | rel. Lebergewicht post | rel. Futterverbrauch post |
|---|---|---|---|---|---|
| 5/ 5 | Formel I; $R^1$, $R^4$, $R^5$ Hydroxyl, $R^2$ und $R^3$ Doppelbindung | 15 | -3 | | -1 |
| 5/ 5 | Lovastatin | 10 | +4 | | +8 |
| 5/ 5 | Lovastatin | 50 | 0 | | +4 |
| 5/ 5 | Lovastatin | 300 | +1 | | +1 |
| 10/10 | Clofibrat | 100 | -2 | +19 | -2 |
| 20/19 | Kontrolle | | 0 | | |

Wie aus Tabelle 1 zu ersehen ist, führt Formel I: $R^1$, $R^4$, $R^5$ Hydroxyl, $R^2$ und $R^3$ Doppelbindung mit nur einem Siebentel der angewandten Dosis von Clofibrat zu einer gleich starken Verminderung der atherogenen Serum-Lipoproteine LDL und VLDL (gemessen anhand ihres Cholesterin-Anteils) und zu einer nur halb so großen Verminderung der HDL-Fraktion. Daraus resultiert eine signifikante Zunahme des vasoprotektiven Index HDL-Cholesterin : LDL-Cholesterin, der ebenfalls deutlich höher als bei Clofibrat liegt. Auch die Triglyceride, die hauptsächlich in den VLDL transportiert werden, sind bei Anwendung der genannten Verbindung deutlich reduziert. Der kompetitive HMG-CoA-Reduktase-Inhibitor Lovastatin ist bezüglich der Beeinflussung von LDL-Cholesterin der erfindungsgemäßen Verbindung deutlich unterlegen, bezüglich der Wirkung auf VLDL und HDL etwa gleich. Die Verträglichkeitsparameter, nämlich die Serum-Enzyme SGOT, SGPT und aP, sowie Serum-Bilirubin und Serum-Kreatinin werden durch erfindungsgemäße Verbindung nicht pathologisch verändert, während Lovastatin dosisabhängig zu einer deutlichen pathologischen Veränderung der Transaminase SGOT führt. Körpergewichtsentwicklung und Futterverbrauch werden durch die geprüften Verbindungen nicht relevant verändert, das relative Lebergewicht wird durch Clofibrat, wie allseits bekannt, erhöht.

2. Biologische Testung als Cholesterinbiosynthese-Inhibitor in Leberzellkulturen

Monolayer von HEP-G2-Zellen in lipoproteinfreiem Nährmedium werden mit entsprechenden Konzentrationen der zu prüfenden Substanzen der allgemeinen Formel I eine Stunde vorinkubiert. Nach Zugabe der $^{14}C$-markierten Biosynthesevorstufe ($^{14}C$)Natriumacetat wird die Inkubation für 3 Stunden fortgesetzt. Danach wird ein Teil der Zellen alkalisch verseift, bei vorheriger Zugabe eines internen Standards von $^{3}H$-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschichtchromatographisch aufgetrennt, die Cholesterinbande nach Anfärbung isoliert und die aus dem $^{14}C$-Precursor gebildete Menge $^{14}C$-Cholesterin szintigraphisch bestimmt. In einem aliquoten Teil der Zellen wurde Zellprotein bestimmt, so daß die in der Zeiteinheit pro mg Zellprotein aus $^{14}C$-Präkursor gebildete Menge $^{14}C$-Cholesterins berechnet werden kann. Zum Vergleich für die Hemmwirkung eines zugesetzten Prüfpräparates dient die Kontrolle, so daß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Lactat-Dehydrogenase-Abgabe ins Inkubationsmedium gemessen.

Als Standardpräparate dienen Lovastatin ($IC_{50}$: 2,3 • $10^{-8}$ molar) und 25-Hydroxycholesterin ($IC_{50}$ 2,3 • $10^{-7}$ molar). Die Verbindung der Formel I, in der $R^1$, $R^4$, $R^5$ Hydroxyl bedeutet und $R^2$ und $R^3$ gemeinsam eine Doppelbindung bilden, hemmt bei 1 • $10^{-7}$ molar die Biosynthese um 50 %; die Zellkultur wird nicht geschädigt.

3. Herstellung von Tabletten

Tabletten, die für die orale Verabreichung geeignet sind und die nachfolgend genannten Bestandteile enthalten, werden auf an sich bekannte Weise hergestellt, indem man Wirk- und Hilfsstoffe granuliert und anschließend zu Tabletten verpreßt. Diese Tabletten eignen sich zur Behandlung von Krankheiten, die auf einem erhöhten Cholesterinspiegel beruhen in einer Dosis von einer Tablette 2-4 mal täglich.

| Bestandteile (pro Tablette) | Gewicht (mg) |
|---|---|
| Formel I: | |
| $R^1$, $R^4$, $R^5$ Hydroxyl, | |
| $R^2$ und $R^3$ Doppelbindung | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

4. Herstellung von Kapseln

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelatinekapseln abfüllt. Diese Kapseln dienen zur Behandlung von Krankheiten, die auf einem erhöhten Cholesterinspiegel beruhen, in einer Dosis von einer Kapsel 2-4 mal täglich.

| Bestandteile (pro Kapsel) | Gewicht (mg) |
|---|---|
| Formel I: | |
| $R^1$, $R^4$, $R^5$ Hydroxyl, | |
| $R^2$ und $R^3$ Doppelbindung | 50 mg |
| Milchzucker | 100 mg |
| Maisstärke | 30 mg |
| Talkum | 3 mg |
| kolloidales Siliziumdioxid | 3 mg |
| Magnesiumstearat | 2 mg |

## Patentansprüche

1. Verwendung der Verbindung der allgemeinen Formel I

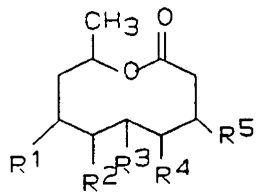

I

in der unabhängig voneinander
$R^1$ und $R^4$ Wasserstoff und Hydroxyl
$R^2$ und $R^3$ Hydroxyl,
$R^5$ Wasserstoff, Hydroxyl und eine Oxogruppe sind und
$R^2$ und $R^3$ sowie $R^3$ und $R^4$ jeweils gemeinsam eine Doppelbindung bilden können,
zur Herstellung eines Arzneimittels zur Senkung des Serum-Cholesterins.

2. Verwendung der Verbindung der Formel I,

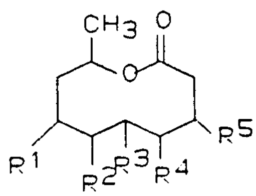

I

in der $R^1$, $R^4$ und $R^5$ Hydroxyl bedeuten und $R^2$ und $R^3$ gemeinsam eine Doppelbindung bilden, zur Herstellung eines Arzneimittels zur Senkung des Serum-Cholesterins.

3. Verwendung der Verbindung der Formel I gemäß Anspruch 1 zur Herstellung eines Arzneimittels zur oralen Anwendung bei der Behandlung des Serum-Cholesterins.

## Claims

1. The use of the compound of the formula I

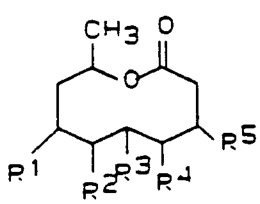

I

in which, independently of one another,
$R^1$ and $R^4$ are hydrogen and hydroxyl,
$R^2$ and $R^3$ are hydroxyl,
$R^5$ is hydrogen, hydroxyl and an oxo group, and
$R^2$ and $R^3$, and $R^3$ and $R^4$, can each together form a double bond,
for the preparation of a pharmaceutical for lowering serum cholesterol.

**2.** The use of the compound of the formula I

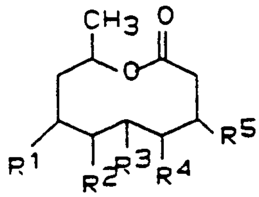

I

in which $R^1$, $R^4$ and $R^5$ are hydroxyl, and $R^2$ and $R^3$ together form a double bond, for the preparation of a pharmaceutical for lowering serum cholesterol.

**3.** The use of the compound of the formula I as claimed in claim 1 for the preparation of a pharmaceutical for oral administration for the treatment of serum cholesterol.

**Revendications**

**1.** Utilisation du composé de formule générale I

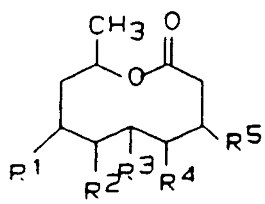

I

dans laquelle, indépendamment les uns des autres,
$R^1$ et $R^4$ représentent un atome d'hydrogène ou le groupe hydroxy,
$R^2$ et $R^3$ représentent le groupe hydroxy,
$R^5$ représente un atome d'hydrogène ou le groupe hydroxy ou oxo, et
$R^2$ et $R^3$ ainsi que $R^3$ et $R^4$ peuvent respectivement former ensemble une double liaison,
pour la fabrication d'un médicament destiné à abaisser le taux du cholestérol sérique.

**2.** Utilisation du composé de formule I

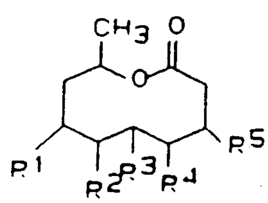

I

dans laquelle $R^1$, $R^4$ et $R^5$ représentent le groupe hydroxy et $R^2$ et $R^3$ forment ensemble une double liaison, pour la fabrication d'un médicament destiné à abaisser le taux de cholestérol sérique.

**3.** Utilisation du composé de formule I selon la revendication 1, pour la fabrication d'un médicament destiné à l'administration orale dans le traitement du cholestérol sérique.